# EUROPEAN PATENT SPECIFICATION

(11) **EP 4 003 343 B1**
(45) Date of publication and mention of the grant of the patent: **04.11.2026**
(21) Application number: 20754480.0
(22) Date of filing: 23.07.2020
(51) Int. Cl.: A61K 31/4412, A61K 31/4166, A61K 31/4745, A61K 33/243, A61K 45/06, A61P 35/00

(54) **THE EZH2 INHIBITOR TULMIMETOSTAT FOR USE IN THE TREATMENT OF SOLID TUMORS**
DER EZH2-HEMMER TULMIMETOSTAT ZUR VERWENDUNG ZUR BEHANDLUNG VON SOLIDEN TUMOREN
L'INHIBITEUR D'EZH2 TULMIMETOSTAT POUR SON UTILISATION DANS LE TRAITEMENT DE TUMEURS SOLIDES

(30) Priority: 24.07.2019 US 201962878021 P
(43) Date of publication of application: 01.06.2022
(73) Proprietor: Constellation Pharmaceuticals, Inc., Boston, MA 02210 (US)
(72) Inventor: BRADLEY, William, D., Winchester, MA 01890 (US)
(74) Representative: Potter Clarkson
(86) International application number: PCT/US2020/043163
(87) International publication number: WO 2021/016409

(56) References cited:
- EP-A1- 3 121 175
- EP-A1- 3 329 917
- WO-A1-2019/204490
- HATTA ET AL: "Development and future prospect EZH1/2 dual inhibitor for malignant lymphoma", HEMATOLOGY / KETSUEKI NAIKA, KAGAKU HYORONSHA, JP, vol. 77, no. 4, 1 January 2018 (2018-01-01), pages 457 - 463, XP009523296, ISSN: 2185-582X

## Description

### BACKGROUND

EZH2 (Enhancer of Zeste Homolog 2) is a histone lysine methyltransferase that has been implicated in the pathogenesis of both hematologic and non-hematologic malignancies. EZH2 catalyzes the transfer of one, two and three methyl-groups to lysine 27 of histone 3 (H3K27). EZH2 is the catalytic component of a large, multi-protein complex called polycomb repressive complex 2 (PRC2), which generally functions in transcriptional repression (Margueron, R., and Reinberg, D. (2011). The Polycomb complex PRC2 and its mark in life. Nature 469, 343-349.). Although in many instances transcriptional silencing by PRC2 is dependent on the catalytic activity of EZH2, it is clear that the physical association of the PRC2 complex with certain genes is also important in transcriptional suppression. The PRC2 complex can alternatively contain a closely related homolog of EZH2, known as EZH1. These two catalytic subunits of the PRC2 complex are the only enzymes known to catalyze H3K27 methylation. In addition to their catalytic activity, EZH1 and EZH2 are multi-domain proteins that mediate other biologic effects through protein-protein and protein-nucleic acid interactions. H3K27 di-methylation and tri-methylation (H3K27me2 and H3K27me3) correlate well with transcriptionally repressed genes, but H3K27 monomethylation (H3K27me1) is found on transcriptionally active genes (Barski, A., et al. (2007). High-resolution profiling of histone methylations in the human genome. Cell 129, 823-837; Ferrari, K.J., et al. (2014). Polycomb-dependent H3K27me1 and H3K27me2 regulate active transcription and enhancer fidelity. Mol. Cell 53, 49-62.). Recent genetic studies suggest that EZH1-containing PRC2 controls H3K27me1 levels (Hidalgo, I., et al. (2012). Ezh1 is required for hematopoietic stem cell maintenance and prevents senescence-like cell cycle arrest. Cell Stem Cell 11, 649-662; Xie, H., et al. (2014). Polycomb repressive complex 2 regulates normal hematopoietic stem cell function in a developmental-stage-specific manner. Cell Stem Cell 14, 68-80.). This is consistent with a putative role of EZH1 in transcriptional elongation (Mousavi, K., et al. (2012). Polycomb protein Ezh1 promotes RNA polymerase II elongation. Mol. Cell 45, 255-262.). Thus, PRC2-dependent H3K27 methyltransferase activity is implicated in both transcriptional repression and activation, depending on the composition of the complex.

EZH2 (but not EZH1) is frequently overexpressed in human cancer. The molecular basis for EZH2 overexpression in cancer includes (1) genomic amplification of the EZH2-encoding gene locus (Tiffen, J., et al. (2016). Somatic Copy Number Amplification and Hyperactivating Somatic Mutations of EZH2 Correlate With DNA Methylation and Drive Epigenetic Silencing of Genes Involved in Tumor Suppression and Immune Responses in Melanoma. Neoplasia 18(2), 121-132., Ding, L., et al. (2006). Identification of EZH2 as a molecular marker for a precancerous state in morphologically normal breast tissues. Cancer Research 66(8), 4095-4099., Saramaki, O.R., et al. (2006). The gene for polycomb group protein enhancer of zeste homolog 2 (EZH2) is amplified in late-stage prostate cancer. Genes Chromosomes Cancer 45(7), 639-645.), (2) deletion and epigenetic silencing of microRNAs that attenuate EZH2 expression (Varambally, S., et al. (2008). Genomic loss of microRNA-101 leads to overexpression of histone methyltransferase EZH2 in cancer. Science 322(5908), 1695-1699.) and (3) dysregulation of gene control exerted by the E2F family of transcription factors (Santos, M., et al. (2014). In vivo disruption of an Rb-E2F-Ezh2 signaling loop causes bladder cancer. Cancer Research 74(22), 6565-6577., Coe, B.P., et al. (2013). Genomic deregulation of the E2F/Rb pathway leads to activation of the oncogene EZH2 in small cell lung cancer. PLoS One 8(8), e71670., Bracken, A.P., et al. (2003). EZH2 is downstream of the pRB-E2F pathway, essential for proliferation and amplified in cancer. EMBO J 22(20), 5323-5335.) such as for instance in contexts of deletion of the *RB1* gene. Thus, there are several recurrent genomic aberrations in cancer that result in EZH2 overexpression, evidencing increased EZH2 levels promote tumor progression. To this end, EZH2 has been linked to a multitude of cancer targets such as hematological malignancies and solid tumors. See e.g., WO 2014/124418.

An EZH2 inhibitor that has gained attention due to its antitumor activity and long residence time in the PRC2 complex (~101 days) is 7-chloro-2-(4-(3-methoxyazetidin-1-yl)cyclohexyl)-2,4-dimethyl-N-((6-methyl-4-(methylthio)-2-oxo-1,2-dihydropyridin-3-yl)methyl)benzo[d][1,3]dioxole-5-carboxamide. See e.g., PCT/US2019/027932. Given its therapeutic potential, and the prevalence of diseases such as cancer, the need exists for alternative therapeutic uses for this compound e.g., for use in combination based treatments. EP 3329917 refers to an agent for treating and/or preventing adult T cell leukemia/lymphoma containing, as an active ingredient, 7-chloro-2-[trans-4-(dimethylamino)cyclohexyl]-N-[(4,6-dimethyl-2-oxo-1,2-dihydropyridin-3-yl)methyl]-2,4-dimethyl-1,3-benzodioxole-5-carboxamide or a pharmaceutically acceptable salt thereof, or 7-bromo-2-[trans-4-(dimethylamino)cyclohexyl]-N-[(4,6-dimethyl-2-oxo-1,2-dihydropyridin-3-yl)methyl]-2,4-dimethyl-1,3-benzodioxole-5-carboxamide or a pharmaceutically acceptable salt thereof. EP3121175 refers to a compound having a 1,3-benzodioxole structure or a pharmacologically acceptable salt thereof which has an inhibitory effect on EZH1 and/or EZH2 activity. Hatta et al. (2018) refers to "Development and future prospect EZH1/2 dual inhibitor for malignant lymphoma".

### SUMMARY

Provided herein is a compound having the formula: 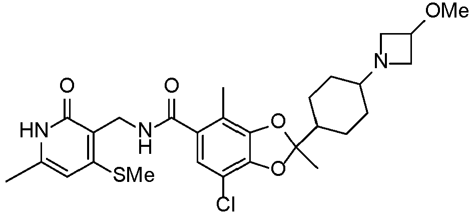 (7-chloro-2-(4-(3-methoxyazetidin-1-yl)cyclohexyl)-2,4-dimethyl-N-((6-methyl-4-(methylthio)-2-oxo-1,2-dihydropyridin-3-yl)methyl)benzo[d][1,3]dioxole-5-carboxamide) or a pharmaceutically acceptable salt thereof for use in the treatment of a solid tumor, wherein the treatment further comprises administration of an effective amount of a second agent selected from a topoisomerase inhibitor, a DNA alkylating agent, and an androgen receptor signaling inhibitor.

Also provided herein is a compound having the formula: 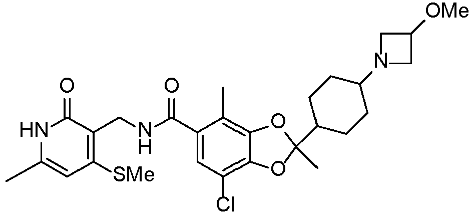 (7-chloro-2-(4-(3-methoxyazetidin-1-yl)cyclohexyl)-2,4-dimethyl-N-((6-methyl-4-(methylthio)-2-oxo-1,2-dihydropyridin-3-yl)methyl)benzo[d][1,3]dioxole-5-carboxamide) or a pharmaceutically acceptable salt thereof, for use in the treatment of an advanced relapsed solid tumor.

Also provided herein are pharmaceutical compositions comprising 7-chloro-2-(4-(3-methoxyazetidin-1-yl)cyclohexyl)-2,4-dimethyl-N-((6-methyl-4-(methylthio)-2-oxo-1,2-dihydropyridin-3-yl)methyl)benzo[d][1,3]dioxole-5-carboxamide; or a pharmaceutically acceptable salt thereof; and a second agent selected from a topoisomerase inhibitor, a DNA alkylating agent, and an androgen receptor signaling inhibitor; and optionally a pharmaceutically acceptable carrier.

### BRIEF DESCRIPTION OF THE FIGURES

**FIG. 1A** shows the phenotypic response of cisplatin sensitive and resistant A2780 (ovarian cancer) and HT1376 (bladder cancer) cell lines to single treatment with cisplatin. Data shown are mean cell viability ± standard error of the mean (SEM), n = 2-3 and are representative of duplicate independent experiments.
**FIG. 1B** shows the phenotypic response of cisplatin sensitive and resistant A2780 (ovarian cancer) and HT1376 (bladder cancer) cell lines to single treatment with Compound **1.** Data shown are mean cell viability ± standard error of the mean (SEM), n = 2-3 and are representative of duplicate independent experiments.
**FIG. 2A** shows the representative growth curves for cisplatin alone and combinations with a dose-titration of Compound **1** in cisplatin sensitive and resistant A2780 ovarian cancer cell lines. Representative of duplicate independent experiments; mean ± SD shown.
**FIG. 2B** shows the combination of sub-GI₅₀ doses for cisplatin and sub-GI₅₀ dose of 16 nM Compound **1** in A2780-P and A2780-CR. Representative of duplicate independent experiments; mean ± SD shown.
**FIG. 3A** shows the representative growth curves for cisplatin alone and combinations with a dose-titration of Compound **1** in cisplatin sensitive and resistant HT1376 bladder cancer cell lines. Representative of duplicate independent experiments; mean ± SD shown.
**FIG. 3B** shows the combination of near-GI₅₀ doses for cisplatin and Compound **1** in HT1376-DMF and HT1376-CR. Representative of duplicate independent experiments; mean ± SD shown.
**FIG. 4** illustrates the antitumor effect of Compound **1,** cisplatin, and the combination of both in HT1376 tumors in CB17 SCID mice. Data shown are mean tumor size ± SEM, n =6. PO = oral administration, IV = intravenous administration, QD = once per day, QW = once per week.
**FIG. 5** illustrates the antitumor effect of Compound **1,** enzalutamide, and the combination of both in CTG-2428 patient-derived xenograft (PDX) model of prostate cancer. Data shown are mean tumor size ± SEM, n=5 per arm. Arrows indicate unscheduled deaths or animal termination due to achievement of maximum tumor volume, n indicates remaining animals per arm. PO = oral administration, QD = once per day.
**FIG. 6** illustrates the antitumor effect of Compound **1,** enzalutamide, and the combination of both in CTG-2440 PDX model of prostate cancer. Data shown are mean tumor size ± SEM, PO = oral administration, QD = once per day. Arrows indicate animal death in combination arm and individual animal taken off stuff due to maximum tumor volume, resulting in reduction of group size, n = remaining animals in arm.
**FIG. 7** illustrates the antitumor effect of Compound **1,** enzalutamide, and the combination of both in CTG-2441 PDX model of prostate cancer. Data shown are mean tumor size ± SEM, n=5. Arrows indicate animal deaths, resulting in reduction of group size, n = remaining animals in arm. PO = oral administration, QD = once per day.

### DETAILED DESCRIPTION

In a first embodiment, provided is a compound having the formula: 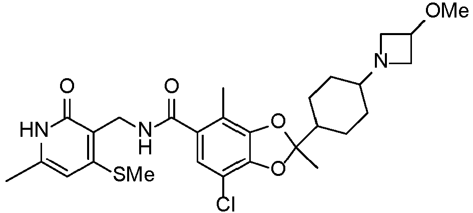 (7-chloro-2-(4-(3-methoxyazetidin-1-yl)cyclohexyl)-2,4-dimethyl-N-((6-methyl-4-(methylthio)-2-oxo-1,2-dihydropyridin-3-yl)methyl)benzo[d][1,3]dioxole-5-carboxamide); or a pharmaceutically acceptable salt thereof; for use in the treatment of a solid tumor, wherein the treatment further comprises administration of an effective amount of second agent selected from a topoisomerase inhibitor and an androgen receptor signaling inhibitor. In an alternative, as part of a first embodiment, provided is an effective amount of 7-chloro-2-(4-(3-methoxyazetidin-1-yl)cyclohexyl)-2,4-dimethyl-N-((6-methyl-4-(methylthio)-2-oxo-1,2-dihydropyridin-3-yl)methyl)benzo[d][1,3]dioxole-5-carboxamide; or a pharmaceutically acceptable salt thereof; and an effective amount of second agent selected from a topoisomerase inhibitor and an androgen receptor signaling inhibitor for use in treating a solid tumor in a subject.

7-chloro-2-(4-(3-methoxyazetidin-1-yl)cyclohexyl)-2,4-dimethyl-N-((6-methyl-4-(methylthio)-2-oxo-1,2-dihydropyridin-3-yl)methyl)benzo[d][1,3]dioxole-5-carboxamide has the chemical formula: 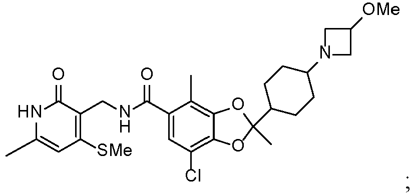 and is disclosed in international application No. PCT/US2019/027932. "Compound 1" is used interchangeably with 7-chloro-2-(4-(3-methoxyazetidin-1-yl)cyclohexyl)-2,4-dimethyl-N-((6-methyl-4-(methylthio)-2-oxo-1,2-dihydropyridin-3-yl)methyl)benzo[d][1,3]dioxole-5-carboxamide and each include stereoisomeric and geometric forms.

Topoisomerase inhibitors of the present invention refer to chemical or biological agents that block the action of topoisomerase (including topoisomerase I and II). As part of a second embodiment, topoisomerase inhibitors of the present invention (e.g., as in the first embodiment) include, but are not limited to, irinotecan, topotecan, camptothecin, lamellarin, etoposide, teniposide, doxorubicin, daunorubicin, mitoxantrone, amsacrine, ellipticines, aurintricarboxylic acid, HU-331, epirubicin, valrubicin, idarubicin, pixantrone, teniposide, belotecan, gimatecan, indotecan, indimitecan. Alternatively, as part of a second embodiment, the topoisomerase inhibitor of the present invention (e.g., as in the first embodiment) is a topoisomerase I inhibitor. In another alternative, as part of a second embodiment, the topoisomerase inhibitor of the present invention (e.g., as in the first embodiment) is irinotecan. In another alternative, as part of a second embodiment, the topoisomerase inhibitor of the present invention (e.g., as in the first embodiment) is topotecan.

DNA alkylating agents of the present invention refer to chemical or biological agents which work by preventing the strands of DNA from linking as they should. As part of a third embodiment, the DNA alkylating agent of the present invention (e.g., as in the first embodiment) is selected from busulfan, cyclophosphamide, bendmustine, carboplatin, chlorambucil, cyclophosphamide, cisplatin, temozolomide, melphalan, carmustine, lomustine, dacarbazine, oxaliplatin, ifosamide, thiotepa, trabectedin, altretamine, mechlorethamine, procarbazine, and streptozocin. Alternatively, as part of a third embodiment, the DNA alkylating agent of the present invention (e.g., as in the first embodiment) is cisplatin.

Androgen receptor signaling inhibitors of the present invention refer to chemical or biological agents which block the androgen receptor (AR) and inhibit or suppress androgen production. As part of a fourth embodiment, the androgen receptor signaling inhibitors of the present invention (e.g., as in the first embodiment) is selected from bicalutamide, enzalutamide, apalutamide, flutamide, nilutamide, darolutamide, and abiraterone acetate (wherein the abiraterone acetate may be included alone or in combination with prednisone). Alternatively, as part of a fourth embodiment, the androgen receptor signaling inhibitors of the present invention (e.g., as in the first embodiment) is enzalutamide. In another alternative, as part of a fourth embodiment, the androgen receptor signaling inhibitors of the present invention (e.g., as in the first embodiment) is abiraterone acetate (wherein the abiraterone acetate may be included alone or in combination with prednisone).

As used herein, the terms "treatment," "treat," and "treating" refer to reversing, alleviating, or inhibiting the progress of a cancer, or one or more symptoms thereof, as described herein.

When used to define a cancer, the term "advanced" as in "advanced cancer" or "advance prostate cancer" means that the recited cancer is unresectable, i.e., the cancer is defined as one that cannot be removed completely through surgery or that the cancer is metastatic, or both. In one aspect, "advanced cancer" means that the cancer is unresectable.

Cancers described herein may also be "relapsed" cancers. The term "relapsed cancer" refers to a cancer which was previously in remission and has returned, or the signs and symptoms of the cancer have returned. Remission includes both partial remission (some or not all signs and symptoms of the cancer have disappeared) and complete remission (all signs and symptoms of the cancer have disappeared, although the cancer may still remain in the body). As such, a cancer that is "advanced relapsed" means that the cancer was in remission and has returned and is unresectable.

Exemplary types of cancer treated by the present invention (e.g., as in the first, second, third, or fourth embodiment) include e.g., adrenal cancer, acinic cell carcinoma, acoustic neuroma, acral lentiginous melanoma, acrospiroma, acute eosinophilic leukemia, acute erythroid leukemia, acute lymphoblastic leukemia, acute megakaryoblastic leukemia, acute monocytic leukemia, acute promyelocytic leukemia, adenocarcinoma, adenoid cystic carcinoma, adenoma, adenomatoid odontogenic tumor, adenosquamous carcinoma, adipose tissue neoplasm, adrenocortical carcinoma, adult T-cell leukemia/lymphoma, aggressive NK-cell leukemia, AIDS-related lymphoma, alveolar rhabdomyosarcoma, alveolar soft part sarcoma, ameloblastic fibroma, anaplastic large cell lymphoma, anaplastic thyroid cancer, angioimmunoblastic T-cell lymphoma, angiomyolipoma, angiosarcoma, astrocytoma, atypical teratoid rhabdoid tumor, B-cell chronic lymphocytic leukemia, B-cell prolymphocytic leukemia, B-cell lymphoma, basal cell carcinoma, biliary tract cancer, bladder cancer, blastoma, bone cancer, Brenner tumor, Brown tumor, Burkitt's lymphoma, breast cancer, brain cancer, carcinoma, carcinoma in situ, carcinosarcoma, cartilage tumor, cementoma, myeloid sarcoma, chondroma, chordoma, choriocarcinoma, choroid plexus papilloma, clear-cell sarcoma of the kidney, craniopharyngioma, cutaneous T-cell lymphoma, cervical cancer, colorectal cancer, Degos disease, desmoplastic small round cell tumor, diffuse large B-cell lymphoma, dysembryoplastic neuroepithelial tumor, dysgerminoma, embryonal carcinoma, endocrine gland neoplasm, endodermal sinus tumor, enteropathy-associated T-cell lymphoma, esophageal cancer, fetus in fetu, fibroma, fibrosarcoma, follicular lymphoma, follicular thyroid cancer, ganglioneuroma, gastrointestinal cancer, germ cell tumor, gestational choriocarcinoma, giant cell fibroblastoma, giant cell tumor of the bone, glial tumor, glioblastoma multiforme, glioma, gliomatosis cerebri, glucagonoma, gonadoblastoma, granulosa cell tumor, gynandroblastoma, gallbladder cancer, gastric cancer, hairy cell leukemia, hemangioblastoma, head and neck cancer, hemangiopericytoma, hematological malignancy, hepatoblastoma, hepatosplenic T-cell lymphoma, Hodgkin's lymphoma, non-Hodgkin's lymphoma, invasive lobular carcinoma, intestinal cancer, kidney cancer, laryngeal cancer, lentigo maligna, lethal midline carcinoma, leukemia, leydig cell tumor, liposarcoma, lung cancer, lymphangioma, lymphangiosarcoma, lymphoepithelioma, lymphoma, acute lymphocytic leukemia, acute myelogenous leukemia, chronic lymphocytic leukemia, liver cancer, small cell lung cancer, non-small cell lung cancer, MALT lymphoma, malignant fibrous histiocytoma, malignant peripheral nerve sheath tumor, malignant triton tumor, mantle cell lymphoma, marginal zone B-cell lymphoma, mast cell leukemia, mediastinal germ cell tumor, medullary carcinoma of the breast, medullary thyroid cancer, medulloblastoma, melanoma, meningioma, merkel cell cancer, mesothelioma, metastatic urothelial carcinoma, mixed Mullerian tumor, mucinous tumor, multiple myeloma, muscle tissue neoplasm, mycosis fungoides, myxoid liposarcoma, myxoma, myxosarcoma, nasopharyngeal carcinoma, neurinoma, neuroblastoma, neurofibroma, neuroma, nodular melanoma, ocular cancer, oligoastrocytoma, oligodendroglioma, oncocytoma, optic nerve sheath meningioma, optic nerve tumor, oral cancer, osteosarcoma, ovarian cancer, Pancoast tumor, papillary thyroid cancer, paraganglioma, pinealoblastoma, pineocytoma, pituicytoma, pituitary adenoma, pituitary tumor, plasmacytoma, polyembryoma, precursor T-lymphoblastic lymphoma, primary central nervous system lymphoma, primary effusion lymphoma, primary peritoneal cancer, prostate cancer, pancreatic cancer, pharyngeal cancer, pseudomyxoma peritonei, renal cell carcinoma, renal medullary carcinoma, retinoblastoma, rhabdomyoma, rhabdomyosarcoma, Richter's transformation, rectal cancer, sarcoma, Schwannomatosis, seminoma, Sertoli cell tumor, sex cord-gonadal stromal tumor, signet ring cell carcinoma, skin cancer, small blue round cell tumors, small cell carcinoma, soft tissue sarcoma, somatostatinoma, soot wart, spinal tumor, splenic marginal zone lymphoma, squamous cell carcinoma, synovial sarcoma, Sezary's disease, small intestine cancer, squamous carcinoma, stomach cancer, T-cell lymphoma, testicular cancer, thecoma, thyroid cancer, transitional cell carcinoma, throat cancer, urachal cancer, urogenital cancer, urothelial carcinoma, uveal melanoma, metastatic castration-resistant prostate cancer, ovarian clear cell carcinoma, uterine cancer, verrucous carcinoma, visual pathway glioma, vulvar cancer, vaginal cancer, Waldenstrom's macroglobulinemia, Warthin's tumor, and Wilms' tumor.

The cancer treated by the present invention (as in the first, second, third, or fourth embodiment) is a solid tumor. As presented herein, solid tumors refer to an abnormal mass of tissue that does not typically contain cysts or liquid areas. Solid tumors may be benign or malignant and are classified by the types of cells that form them. Examples of solid tumors include e.g., sarcomas, carcinomas, and lymphomas.

In one aspect, as part of a sixth embodiment, the cancer treated by the present invention (e.g., as in the first, second, third, or fourth embodiment) is a solid malignant tumor. Alternatively, as part of a fifth embodiment, the solid tumor treated by the present invention (e.g., as in the first, second, third, or fourth embodiment) may be selected from bladder cancer, breast cancer, cervical cancer, colon cancer, rectal cancer, uterine cancer, kidney cancer, lip cancer, oral cancer, liver cancer, skin cancer, lung cancer, ovarian cancer, pancreatic cancer, prostate cancer, and gastric or gastroesophageal cancer. In another alternative, as part of a sixth embodiment, the solid tumor treated by the present invention (e.g., as in the first, second, or third embodiment) is selected from prostate cancer, small cell lung cancer (SCLC), gastric or gastroesophageal junction (GEJ) adenocarcinoma, and serous ovarian cancer. In another alternative, as part of a sixth embodiment, the solid tumor treated by the present invention (e.g., as in the first, second, third, or fourth embodiment) is selected from small cell lung cancer (SCLC), gastric or gastroesophageal junction (GEJ) adenocarcinoma, and serous ovarian cancer. In another alternative, as part of a sixth embodiment, the solid tumor treated by the present invention (e.g., as in the first, second, third, or fourth embodiment) is prostate cancer. In another alternative, as part of a sixth embodiment, the solid tumor treated by the present invention (e.g., as in the first, second, third, or fourth embodiment) is selected from urothelial carcinoma, ovarian clear cell carcinoma, and endometrial carcinoma

In one aspect, as part of a seventh embodiment, the cancers treated by the present invention (e.g., as in the first through sixth embodiments) are relapsed cancers. Therefore, as part of a sixth embodiment, the cancers treated by the present invention (e.g., as in the first through sixth embodiments) are relapsed solid tumors such as relapsed prostate cancer, relapsed small cell lung cancer (SCLC), relapsed gastric or gastroesophageal junction (GEJ) adenocarcinoma, and relapsed serous ovarian cancer.

In one aspect, the cancers described herein (e.g., as in the fourth through seventh embodiments) are advanced cancers e.g., advanced prostate cancer, advanced small cell lung cancer (SCLC), advanced gastric or gastroesophageal junction (GEJ) adenocarcinoma, and advanced serous ovarian cancer.

Unless otherwise indicated, the administrations described herein include administering 7-chloro-2-(4-(3-methoxyazetidin-1-yl)cyclohexyl)-2,4-dimethyl-N-((6-methyl-4-(methylthio)-2-oxo-1,2-dihydropyridin-3-yl)methyl)benzo[d][1,3]dioxole-5-carboxamide prior to, concurrently with, or after administration of a disclosed topoisomerase inhibitor, a DNA alkylating agent, or androgen receptor signaling inhibitor described herein (e.g., as in the first, second, third, or fourth embodiment) to treat a recited solid tumor (e.g., as in the fifth through seventh embodiments). Thus, simultaneous administration is not necessary for therapeutic purposes. In one aspect, however, 7-chloro-2-(4-(3-methoxyazetidin-1-yl)cyclohexyl)-2,4-dimethyl-N-((6-methyl-4-(methylthio)-2-oxo-1,2-dihydropyridin-3-yl)methyl)benzo[d][1,3]dioxole-5-carboxamide is administered concurrently with the topoisomerase inhibitor, DNA alkylating agent, or androgen receptor signaling inhibitor.

In an eighth embodiment, provided is an effective amount of 7-chloro-2-(4-(3-methoxyazetidin-1-yl)cyclohexyl)-2,4-dimethyl-N-((6-methyl-4-(methylthio)-2-oxo-1,2-dihydropyridin-3-yl)methyl)benzo[d][1,3]dioxole-5-carboxamide, or a pharmaceutically acceptable salt thereof for use in treating advanced relapsed solid tumors in a subject.

Advanced relapsed solid tumors described herein (e.g., those in the seventh embodiment) include, but are not limited to, advanced relapsed urothelial carcinoma, advanced relapsed ovarian clear cell carcinoma, and advanced relapsed endometrial carcinoma.

In a ninth embodiment, provided herein are pharmaceutical compositions comprising an effective amount of 7-chloro-2-(4-(3-methoxyazetidin-1-yl)cyclohexyl)-2,4-dimethyl-N-((6-methyl-4-(methylthio)-2-oxo-1,2-dihydropyridin-3-yl)methyl)benzo[d][1,3]dioxole-5-carboxamide, or a pharmaceutically acceptable salt thereof; and an effective amount of second agent selected from a topoisomerase inhibitor and an androgen receptor signaling inhibitor; and optionally a pharmaceutically acceptable carrier. The use of the pharmaceutical composition comprising an effective amount of 7-chloro-2-(4-(3-methoxyazetidin-1-yl)cyclohexyl)-2,4-dimethyl-N-((6-methyl-4-(methylthio)-2-oxo-1,2-dihydropyridin-3-yl)methyl)benzo[d][1,3]dioxole-5-carboxamide, or a pharmaceutically acceptable salt thereof; and an effective amount of second agent selected from a topoisomerase inhibitor and an androgen receptor signaling inhibitor; and optionally a pharmaceutically acceptable carrier for treating one or more solid tumors described herein (e.g., as in the fifth through seventh embodiment) is also included. Further provided in the use of a pharmaceutical composition comprising 7-chloro-2-(4-(3-methoxyazetidin-1-yl)cyclohexyl)-2,4-dimethyl-N-((6-methyl-4-(methylthio)-2-oxo-1,2-dihydropyridin-3-yl)methyl)benzo[d][1,3]dioxole-5-carboxamide, or a pharmaceutically acceptable salt thereof for treating an advanced relapsed solid tumor.

In one aspect, 7-chloro-2-(4-(3-methoxyazetidin-1-yl)cyclohexyl)-2,4-dimethyl-N-((6-methyl-4-(methylthio)-2-oxo-1,2-dihydropyridin-3-yl)methyl)benzo[d][1,3]dioxole-5-carboxamide is of crystalline Form 1 characterized by at least three X-ray powder diffraction peaks at 2Θ angles selected from 10.0°, 13.3°, 14.9°, 20.2°, 20.8°, 22.2°, and 22.5°. Alternatively, 7-chloro-2-(4-(3-methoxyazetidin-1-yl)cyclohexyl)-2,4-dimethyl-N-((6-methyl-4-(methylthio)-2-oxo-1,2-dihydropyridin-3-yl)methyl)benzo[d][1,3]dioxole-5-carboxamide is of crystalline Form 1 characterized by at least four X-ray powder diffraction peaks at 2Θ angles selected from 10.0°, 13.3°, 14.9°, 20.2°, 20.8°, 22.2°, and 22.5°. In another alternative, 7-chloro-2-(4-(3-methoxyazetidin-1-yl)cyclohexyl)-2,4-dimethyl-N-((6-methyl-4-(methylthio)-2-oxo-1,2-dihydropyridin-3-yl)methyl)benzo[d][1,3]dioxole-5-carboxamide is of crystalline Form 1 characterized by at least five X-ray powder diffraction peaks at 2Θ angles selected from 10.0°, 13.3°, 14.9°, 20.2°, 20.8°, 22.2°, and 22.5°. In another alternative, 7-chloro-2-(4-(3-methoxyazetidin-1-yl)cyclohexyl)-2,4-dimethyl-N-((6-methyl-4-(methylthio)-2-oxo-1,2-dihydropyridin-3-yl)methyl)benzo[d][1,3]dioxole-5-carboxamide is of crystalline Form **1** characterized by at least six X-ray powder diffraction peaks at 2Θ angles selected from 10.0°, 13.3°, 14.9°, 20.2°, 20.8°, 22.2°, and 22.5°. In another alternative, 7-chloro-2-(4-(3-methoxyazetidin-1-yl)cyclohexyl)-2,4-dimethyl-N-((6-methyl-4-(methylthio)-2-oxo-1,2-dihydropyridin-3-yl)methyl)benzo[d][1,3]dioxole-5-carboxamide is of crystalline Form **1** characterized by X-ray powder diffraction peaks at 2Θ angles selected from 10.0°, 13.3°, 14.9°, 20.2°, 20.8°, 22.2°, and 22.5°. In another alternative, 7-chloro-2-(4-(3-methoxyazetidin-1-yl)cyclohexyl)-2,4-dimethyl-N-((6-methyl-4-(methylthio)-2-oxo-1,2-dihydropyridin-3-yl)methyl)benzo[d][1,3]dioxole-5-carboxamide is of crystalline Form **1** characterized by X-ray powder diffraction peaks at 2Θ angles selected from 10.0°, 10.2°, 12.3°, 12.7°, 13.3°, 14.9°, 15.3°, 20.2°, 20.8°, 21.3°, 22.2°, 22.5°, and 23.8°. In another alternative, 7-chloro-2-(4-(3-methoxyazetidin-1-yl)cyclohexyl)-2,4-dimethyl-N-((6-methyl-4-(methylthio)-2-oxo-1,2-dihydropyridin-3-yl)methyl)benzo[d][1,3]dioxole-5-carboxamide is of crystalline Form **1** characterized by X-ray powder diffraction peaks at 2Θ angles selected from 10.0°, 10.2°, 11.0°, 11.4°, 11.8°, 12.3°, 12.7°, 13.3°, 14.9°, 15.3°, 16.1°, 17.4°, 20.2°, 20.8°, 21.3°, 22.2°, 22.5°, and 23.8°. In another alternative, 7-chloro-2-(4-(3-methoxyazetidin-1-yl)cyclohexyl)-2,4-dimethyl-N-((6-methyl-4-(methylthio)-2-oxo-1,2-dihydropyridin-3-yl)methyl)benzo[d][1,3]dioxole-5-carboxamide is of crystalline Form **1** characterized by x-ray powder diffraction peaks at 2Θ angles selected from 14.9°, 20.2°, and 20.8°. In another alternative, 7-chloro-2-(4-(3-methoxyazetidin-1-yl)cyclohexyl)-2,4-dimethyl-N-((6-methyl-4-(methylthio)-2-oxo-1,2-dihydropyridin-3-yl)methyl)benzo[d][1,3]dioxole-5-carboxamide is of crystalline Form **1** characterized by x-ray powder diffraction peaks at 2Θ angles selected from 10.0°, 14.9°, 20.2°, and 20.8°. In another alternative, 7-chloro-2-(4-(3-methoxyazetidin-1-yl)cyclohexyl)-2,4-dimethyl-N-((6-methyl-4-(methylthio)-2-oxo-1,2-dihydropyridin-3-yl)methyl)benzo[d][1,3]dioxole-5-carboxamide is of crystalline Form **1** characterized by x-ray powder diffraction peaks at 2Θ angles selected from 10.0°, 14.9°, 20.2°, 20.8°, and 22.2°. In another alternative, 7-chloro-2-(4-(3-methoxyazetidin-1-yl)cyclohexyl)-2,4-dimethyl-N-((6-methyl-4-(methylthio)-2-oxo-1,2-dihydropyridin-3-yl)methyl)benzo[d][1,3]dioxole-5-carboxamide is of crystalline Form **1** characterized by x-ray powder diffraction peaks at 2Θ angles selected from 10.0°, 13.3°, 14.9°, 20.2°, 20.8°, and 22.2°.

In one aspect, the 7-chloro-2-(4-(3-methoxyazetidin-1-yl)cyclohexyl)-2,4-dimethyl-N-((6-methyl-4-(methylthio)-2-oxo-1,2-dihydropyridin-3-yl)methyl)benzo[d][1,3]dioxole-5-carboxamide described herein is (2R)-7-chloro-2-(trans-4-(3-methoxyazetidin-1-yl)cyclohexyl)-2,4-dimethyl-N-((6-methyl-4-(methylthio)-2-oxo-1,2-dihydropyridin-3-yl)methyl)benzo[d][1,3]dioxole-5-carboxamide.

The term "pharmaceutically acceptable carrier" refers to a non-toxic carrier, adjuvant, or vehicle that does not adversely affect the pharmacological activity of the compound with which it is formulated, and which is also safe for human use. Pharmaceutically acceptable carriers, adjuvants or vehicles that may be used in the compositions of this disclosure include, but are not limited to, ion exchangers, alumina, aluminum stearate, magnesium stearate, lecithin, serum proteins, such as human serum albumin, buffer substances such as phosphates, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinyl pyrrolidone, cellulose-based substances (e.g., microcrystalline cellulose, hydroxypropyl methylcellulose, lactose monohydrate, sodium lauryl sulfate, and crosscarmellose sodium), polyethylene glycol, sodium carboxymethylcellulose, polyacrylates, waxes, polyethylene-polyoxypropylene-block polymers, polyethylene glycol and wool fat.

The compounds described herein may be present in the form of pharmaceutically acceptable salts. For use in medicines, the salts of the compounds described herein refer to non-toxic "pharmaceutically acceptable salts." Pharmaceutically acceptable salt forms include pharmaceutically acceptable acidic/anionic or basic/cationic salts where possible.

Compositions and compounds provided herein may be administered orally, parenterally, by inhalation spray, topically, rectally, nasally, buccally, vaginally or via an implanted reservoir. The term "parenteral" as used herein includes subcutaneous, intravenous, intramuscular, intra-articular, intra-synovial, intrasternal, intrathecal, intrahepatic, intralesional and intracranial injection or infusion techniques.

It should also be understood that a specific dosage and treatment regimen for any particular patient will depend upon a variety of factors, including age, body weight, general health, sex, diet, time of administration, rate of excretion, drug combination, the judgment of the treating physician, and the severity of the particular disease being treated. The amount of a provided compound in the composition will also depend upon the particular compound in the composition.

The terms "subject" and "patient" may be used interchangeably, and means a mammal in need of treatment, *e.g.,* companion animals (*e.g.,* dogs, cats, and the like), farm animals (*e.g.,* cows, pigs, horses, sheep, goats and the like) and laboratory animals (*e.g.,* rats, mice, guinea pigs and the like). Typically, the subject is a human in need of treatment.

The term "effective amount" or "therapeutically effective amount" refers to an amount of a compound described herein that will elicit a biological or medical response of a subject e.g., a dosage of between 0.01 - 100 mg/kg body weight/day. In one aspect, the effective amount of 7-chloro-2-(4-(3-methoxyazetidin-1-yl)cyclohexyl)-2,4-dimethyl-N-((6-methyl-4-(methylthio)-2-oxo-1,2-dihydropyridin-3-yl)methyl)benzo[d][1,3]dioxole-5-carboxamide, or a pharmaceutically acceptable salt thereof; and the effective amount of a topoisomerase inhibitor, DNA alkylating agent, or androgen receptor signaling inhibitor described herein is such that together, they elicit a combinatorial effect to measurably treat one or more solid tumors described herein.

### EXEMPLIFICATION

The present invention will now be illustrated by the following non-limiting examples.

Compound **1** can be prepared as a single enantiomer, single geometric isomer, using the following procedure below.

### Intermediate 1: methyl 7-chloro-2,4-dimethyl-2-(4-oxocyclohexyl)benzo[d] [1,3]dioxole-5-carboxylate

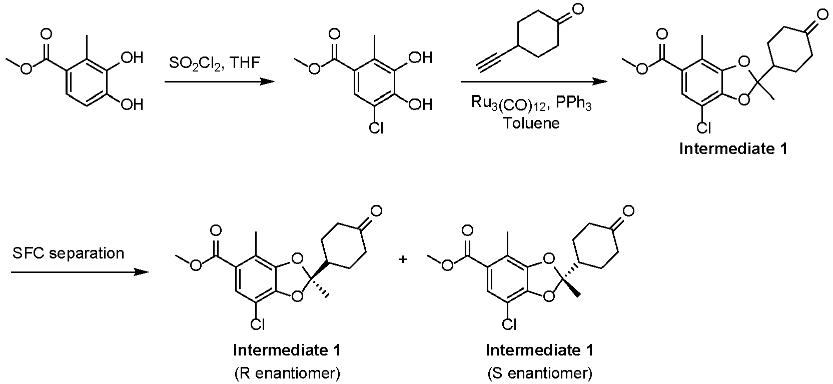

### Step 1: Synthesis of methyl 5-chloro-3,4-dihydroxy-2-methylbenzoate

To a solution of methyl 3,4-dihydroxy-2-methylbenzoate (5.11 g, 27.9 mmol) in tetrahydrofuran (199 mL) at -20 °C was added sulfuryl chloride (2.45 mL, 30.6 mmol) dropwise. The reaction mixture was stirred at -20 °C for 3 h then quenched with a saturated aqueous solution of ammonium chloride (50 mL). The desired product was extracted with ethyl acetate (25 mL × 3). The combined organic layers were washed with brine (25 mL), dried over sodium sulfate, filtered, and concentrated to dryness under reduced pressure. The residue was purified by flash chromatography (silica gel, gradient 0% to 60% ethyl acetate in heptane) to give the title compound (4.117 g, 68% yield) as a beige solid. LCMS [M+H]⁺ m/z: calc'd 217.0; found 217.1 (Cl isotope pattern).

### Step 2: Synthesis of methyl 7-chloro-2,4-dimethyl-2-(4-oxocyclohexyl)-2H-1,3-benzodioxole-5-carboxylate

A mixture of methyl 5-chloro-3,4-dihydroxy-2-methylbenzoate (1.2 g, 5.53 mmol), triruthenium dodecacarbonyl (176 mg, 276 µmol), and triphenylphosphine (145 mg, 553 µmol) was degassed under vacuum and purged with nitrogen (3 cycles). Toluene (8.1 mL) was added and the reaction mixture was heated to reflux for 30 min. A solution of 4-ethynylcyclohexan-1-one (1.34 g, 11.0 mmol) in toluene (17 mL) was then added dropwise and the reaction stirred for 23 h at reflux. Finally, the reaction mixture was cooled to room temperature and concentrated to dryness under reduced pressure. The residue was purified by flash chromatography (silica gel, gradient 0 to 60% ethyl acetate in heptane) to give the title compound (1.327 g, 70% yield) as a yellow oil. LCMS [M+Na]⁺ m/z: calc'd 361.1; found 361.1 (Cl isotope pattern).

### Step 3: Separation of methyl (R)-7-chloro-2,4-dimethyl-2-(4-oxocyclohexyl)benzo[d][1,3]dioxole-5-carboxylate and methyl (S)-7-chloro-2,4-dimethyl-2-(4-oxocyclohexyl)benzo[d][1,3]dioxole-5-carboxylate

The racemic mixture of methyl-7-chloro-2,4-dimethyl-2-(4-oxocyclohexyl)benzo[d][1,3]dioxole-5-carboxylate (4.4 g, 13 mmol) was resolved by preparative SFC [Column: ChiralPak AY from Daicel chemical industries (250mm×50mm I.D., 10µm). Mobile phase A: CO₂ / Mobile phase B: 0.1% NH₄OH in methanol. Isocratic (85% mobile phase A and 15% mobile phase B). Flow rate: 80 mL/min. Column temperature: 40 °C]. Intermediate 1 (Peak 1) (undesired enantiomer/distomer): Retention time = 6.2 min. Recovery = 1.4 g, 4.05 mmol, 31% yield, 90% ee, 98% purity (yellow solid). ¹H NMR (400 MHz, Chloroform-*d*) δ 7.48 (s, 1H), 3.78 (s, 3H), 2.44 - 2.36 (m, 2H), 2.35 - 2.25 (m, 6H), 2.19 (tdd, *J* = 2.8, 5.6, 13.1 Hz, 2H), 1.70 - 1.57 (m, 5H). Intermediate 1 (Peak 2) (desired enantiomer/eutomer): Retention time = 7.0 min. Recovery = 1.1 g, 3.08 mmol, 23.75% yield, 99% ee, 95% purity (yellow solid). ¹H NMR (400 MHz, Chloroform-d) δ 7.49 (s, 1H), 3.78 (s, 3H), 2.44 - 2.36 (m, 2H), 2.36 - 2.25 (m, 6H), 2.20 (tdd, *J* = 2.8, 5.6, 13.1 Hz, 2H), 1.72 - 1.59 (m, 5H). SFC analytical method: [Column: ChiralPak AY-3 (150×4.6mm I.D., 3µm). Mobile phase A: CO₂ / Mobile phase B: 0.05% Et₂NH in iPrOH. Gradient: from 5 to 40% of mobile phase B (over 5.5 min). Flow rate: 2.5 mL/min. Column temperature: 40 °C]. Intermediate 1 (Peak 1 - undesired enantiomer/ distomer): Retention time = 2.853 min. Intermediate 1 (Peak 2 - desired enantiomer/eutomer): Retention time = 2.979 min.

### Intermediate 2: 7-chloro-2-(4-(3-methoxyazetidin-1-yl)cyclohexyl)-2,4-dimethylbenzo[d][1,3]dioxole-5-carboxylic acid

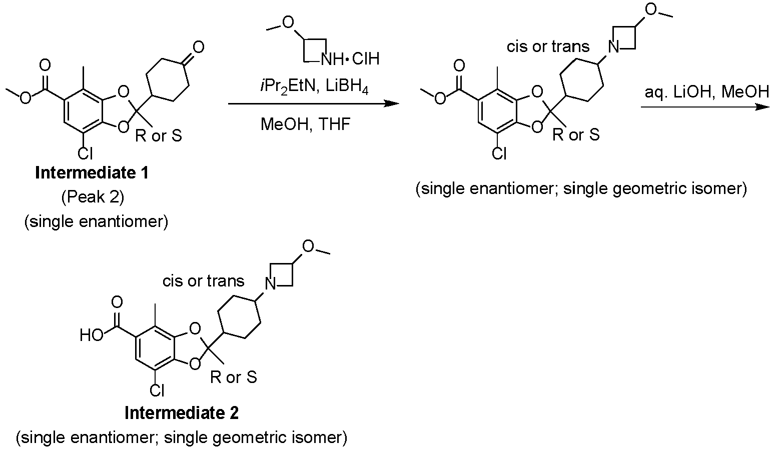

### Step 1: Synthesis of methyl 7-chloro-2-(4-(3-methoxyazetidin-1-yl)cyclohexyl)-2,4-dimethylbenzo[d][1,3]dioxole-5-carboxylate

A solution of 3-methoxyazetidine hydrochloride salt (8 g, 64.75 mmol) and N,N-diisopropylethylamine (12 mL, 68.9 mmol) in methanol (30 mL) was stirred at room temperature for 30 min before a solution of another solution of methyl 7-chloro-2,4-dimethyl-2-(4-oxocyclohexyl)-1,3-benzodioxole-5-carboxylate (Intermediate 1 - Peak 2) (4.1 g, 12.10 mmol) in tetrahydrofuran (30 mL) was added. The reaction mixture was stirred at room temperature for 1 h then cooled to -70 °C. Lithium borohydride (500 mg, 22.96 mmol) was added and the reaction stirred at -70 °C for 30 min [or until complete consumption of the starting material was observed by TLC, ethyl acetate/methanol 5:1]. Next, two batches of the reaction were combined and quenched with a saturated aqueous solution of ammonium chloride (120 mL) at 0 °C and the desired product was extracted with dichloromethane (200 mL × 3). The combined organic layers were dried over sodium sulfate, filtered and concentrated to dryness under reduced pressure. The residue was purified by flash chromatography (silica gel, gradient 0 to 14% methanol in dichloromethane) to give title compound (8.05 g, 67% yield, 83% purity) as a light yellow oil. A sample (50 mg) was purified further by preparative thin layer chromatography (silica gel, ethyl acetate:methanol 15:1). LCMS [M+H]⁺ m/z: calc'd. 410.2; found 410.1. ¹H NMR (400 MHz, Methanol-*d₄*) δ 7.39 (s, 1H), 3.95 - 3.91 (m, 1H), 3.73 (s, 3H), 3.59 - 3.51 (m, 2H), 3.16 (s, 3H), 2.97 (br dd, *J* = 6.4, 8.0 Hz, 2H), 2.26 (s, 3H), 2.11 - 2.02 (m, 1H), 1.91 - 1.73 (m, 5H), 1.54 (s, 3H), 1.22 - 1.12 (m, 2H), 0.98 - 0.86 (m, 2H).

### Step 2: Synthesis of 7-chloro-2-(4-(3-methoxyazetidin-1-yl)cyclohexyl)-2,4-dimethylbenzo[d][1,3]dioxole-5-carboxylic acid

To a solution of methyl 7-chloro-2-(4-(3-methoxyazetidin-1-yl)cyclohexyl)-2,4-dimethylbenzo[d][1,3]dioxole-5-carboxylate (4 g, 9.75 mmol) in methanol (48 mL) was added a solution of lithium hydroxide hydrate (4.03 g, 96.06 mmol) in water (12 mL). The reaction was stirred at 70 °C for 2 h then two batches were combined and concentrated under reduced pressure. Water (50 mL) was added and the pH adjusted to 6 with a saturated aqueous citric acid solution at 0°C. The desired product was extracted with a 3:1 mixture of dichloromethane and isopropanol (300 mL × 5). The combined organic layers were dried over sodium sulfate, filtered and concentrated to dryness under reduced pressure to give the title compound (6.1g, crude) as a off-white solid, which was used in the next step without further purification. LCMS [M+H]⁺ m/z: calc'd. 396.2; found 396.1. ¹H NMR (400 MHz, Methanol-*d*₄) δ 7.07 (s, 1H), 4.05 - 4.10 (m, 2H), 3.76 - 3.88 (m, 1H), 3.67 (br dd, *J =* 10, 3.6 Hz, 2H), 3.22 (s, 3H), 2.71 - 2.81 (m, 1H), 2.19 (s, 3H), 1.91 - 1.99 (m, 4H), 1.75 - 1.85 (m, 1H), 1.52 (s, 3H), 1.18 - 1.28 (m, 2H), 1.06 - 1.14 (m, 2H).

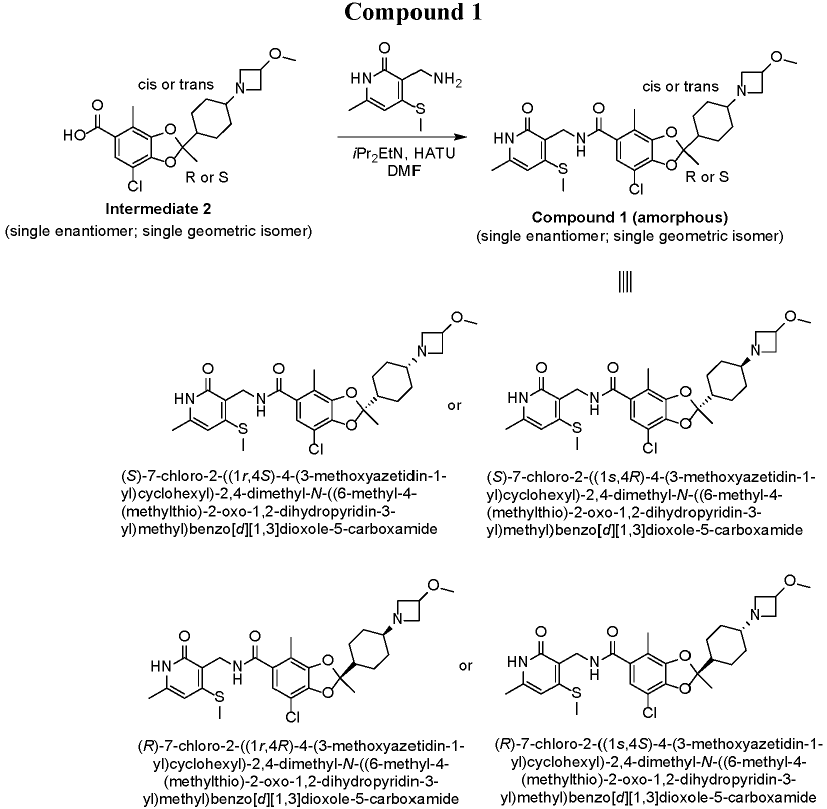

To a solution of 7-chloro-2-(4-(3-methoxyazetidin-1-yl)cyclohexyl)-2,4-dimethylbenzo[d][1,3]dioxole-5-carboxylic acid (Intermediate 2 - single enantiomer and geometric isomer) (5 g, 12.63 mmol) in N,N-dimethylformamide (50 mL) were added O-(7-azabenzotriazol-1-yl)-N,N,N',N'-tetramethyluronium hexafluorophosphate (5.7 g, 14.99 mmol) and N,N-diisopropylethylamine (11 mL, 63.15 mmol). The mixture was stirred at 20 °C for 30 min before 3-(aminomethyl)-6-methyl-4-(methylthio)pyridin-2(1H)-one hydrochloride salt (Intermediate 1) (4.2 g, 19.03 mmol) was added. The reaction mixture was stirred at room temperature for an additional 1.5 h then filtered. The filtrate was purified by preparative HPLC [Column: Phenomenex Gemini C18 (250mm×50mm, 10µm). Mobile phase A: water (0.04% ammonia hydroxide v/v and 10 mM ammonium bicarbonate) / Mobile phase B: acetonitrile. Gradient (75 to 44% mobile phase A / 25 to 56% mobile phase B, over 23 min). Column temperature: 30 °C] to give the title compound (4.4 g, 60% yield, 96% purity as a white solid. LCMS [M+H]⁺ m/z: calc'd.562.2; found 562.2. ¹H NMR (400 MHz, Methanol-*d*₄) δ 6.91 (s, 1H), 6.29 (s, 1H), 4.50 (s, 2H), 4.01 (quin, *J =* 6 Hz, 1H), 3.58 (dd, *J* = 8.8, 6.4 Hz, 2H), 3.26 (s, 3H), 2.92 - 3.02 (m, 2H), 2.54 (s, 3H), 2.31 (s, 3H), 2.21 (s, 3H), 2.01 - 2.11 (m, 1H), 1.79 - 2.00 (m, 5H), 1.62 (s, 3H), 1.19 - 1.34 (m, 2H), 0.91 - 1.08 (m, 2H).

### 1. Primary In Vitro Pharmacology

### A. Mechanism of Action

In biochemical assays, Compound **1** suppresses catalytic activity of wild-type and Y641N mutant EZH2-containing PRC2 complex, as well as EZH1-containing PRC2 complex, with half-maximal inhibitory concentrations (IC50) values of 0.02 and 0.03 nM for wild-type and Y641N mutant EZH2, respectively, and 0.06 nM for EZH1. See e.g., PCT/US2019/027932. The biochemical potency underestimates the true affinity of Compound **1** and further characterization of binding via kinetic assays supports an inhibition constant of approximately 0.11 pM for EZH2 and approximately 70 fold selectivity for EZH2 over EZH1. Based-upon the kinetic analysis it was determined that Compound **1** binds to PRC2 with a long residence time (approximately 101 days). See e.g., PCT/US2019/027932.

### B. Effects on Global H3K27me3 Intracellular Levels and Gene Expression

The ability of Compound **1** to reduce global H3K27me3 intracellular levels was assessed in a wild-type EZH2-containing cervical cancer cell line (HeLa). After 4 days of treatment, Compound **1** was able to reduce global levels of H3K27me3 with an EC50 of 0.40 nM. See e.g., PCT/US2019/027932. Compound **1** was able to exhibit similar potency in other solid tumor cell lines, including bladder cancer (639V and HT1197) and ovarian cancer TOV21G cell lines, with Day 3 EC50 values of 0.09, 0.14 and 0.26 nM, respectively.

Reduction in H3K27me3 levels result in changes in gene expression. RNA-sequencing of bladder cancer cell lines after treatment with Compound **1** for 4 days results in significant changes in the expression levels of multiple genes. The predominant alteration was an increase in gene expression, as very few genes were significantly decreased. The increase in gene expression is both dose and time dependent, with increasing expression observed at higher concentrations of Compound **1** and at later timepoints. This contrasts with reductions of H3K27me3 as methyl mark changes were observed after 1 day of Compound **1** treatment. Of note, one of the genes highly upregulated was CDKN1C, also known as p57 or Kip2, a known tumor suppressor and negative regulator of the cell cycle that has been previously reported to be an EZH2 target gene. See Yang X, Karuturi RK, Sun F, et al. CDKN1C (p57) is a direct target of EZH2 and suppressed by multiple epigenetic mechanisms in breast cancer cells. PLoS One. 2009;4(4):e5011. Low expression of CDKN1C is seen in advanced bladder and breast cancers and is correlated with poor prognosis. See Yang above and Hoffmann MJ, Florl AR, Seifert HH, et al. Multiple mechanisms downregulate CDKN1C in human bladder cancer. Int J Cancer. 2005 Apr 10;114(3):406-13.

### 2. Anti-Proliferative Effects

### A. Synergy With Compound 1 and Cisplatin (DNA Alkylating Agent)

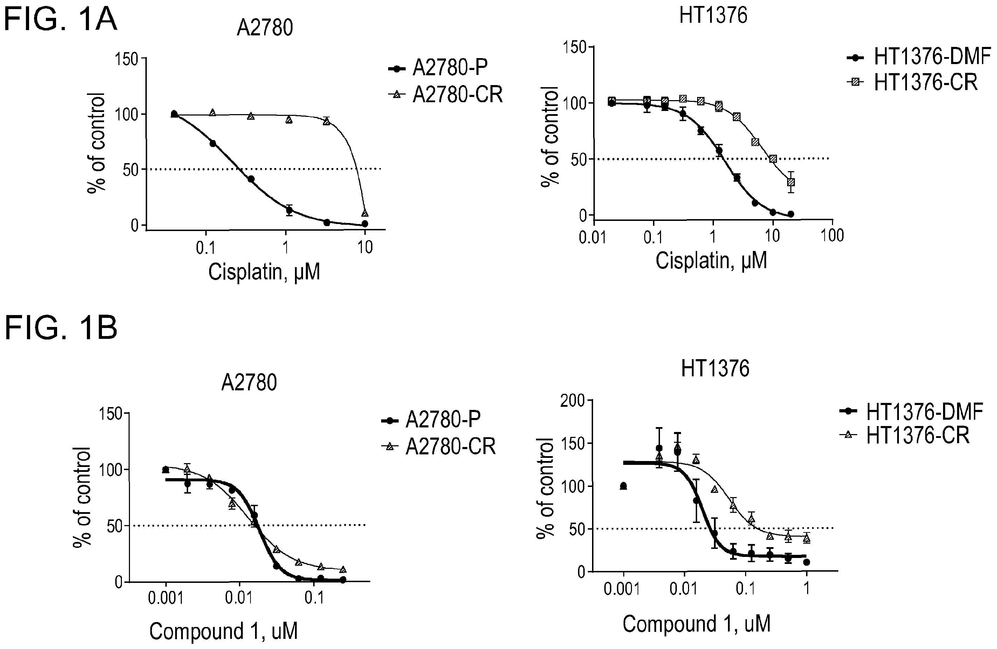

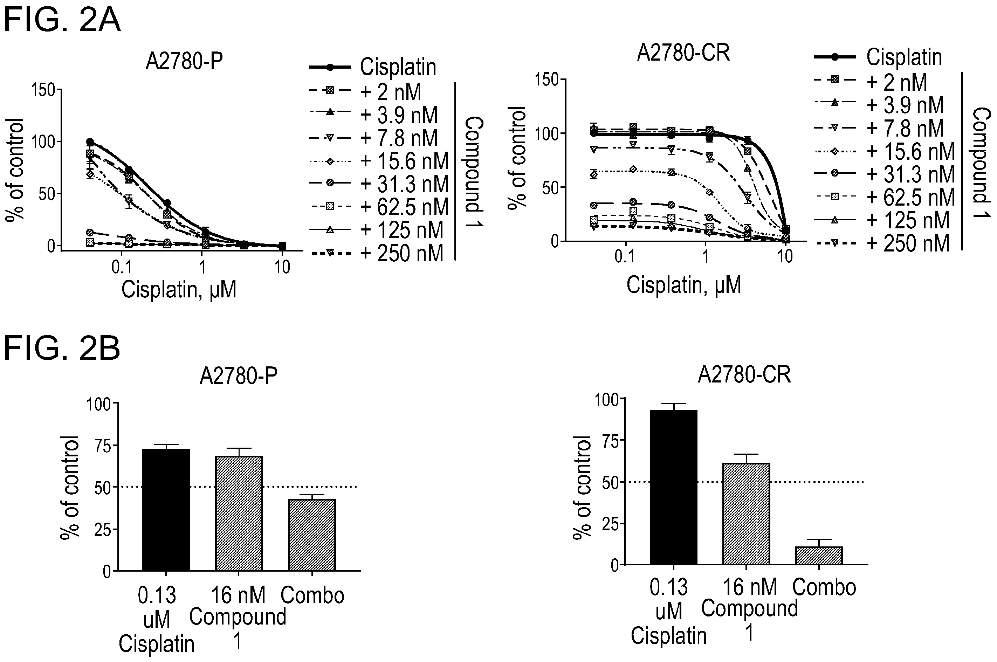

The sensitivity of multiple solid tumor cancer cell lines to the antiproliferative activity of Compound 1 with and without cisplatin was evaluated. We first found that cisplatin resistance versions of ovarian cancer cell line A2780 (A2780-CR) and bladder cancer cell line (HT1376-CR) are less sensitive to cisplatin than parental (A2780-P) or age-matched DMF control (HT1376-DMF) cell lines (See **FIG. 1A****),** while cisplatin resistant versions of A2780 and HT1376 remain strongly sensitive to Compound **1 (****FIG. 1B****).** Combination treatment with Compound **1** and cisplatin, however, led to greater than 50% reduction in growth. See **FIG. 2A** and **FIG. 2B****.**

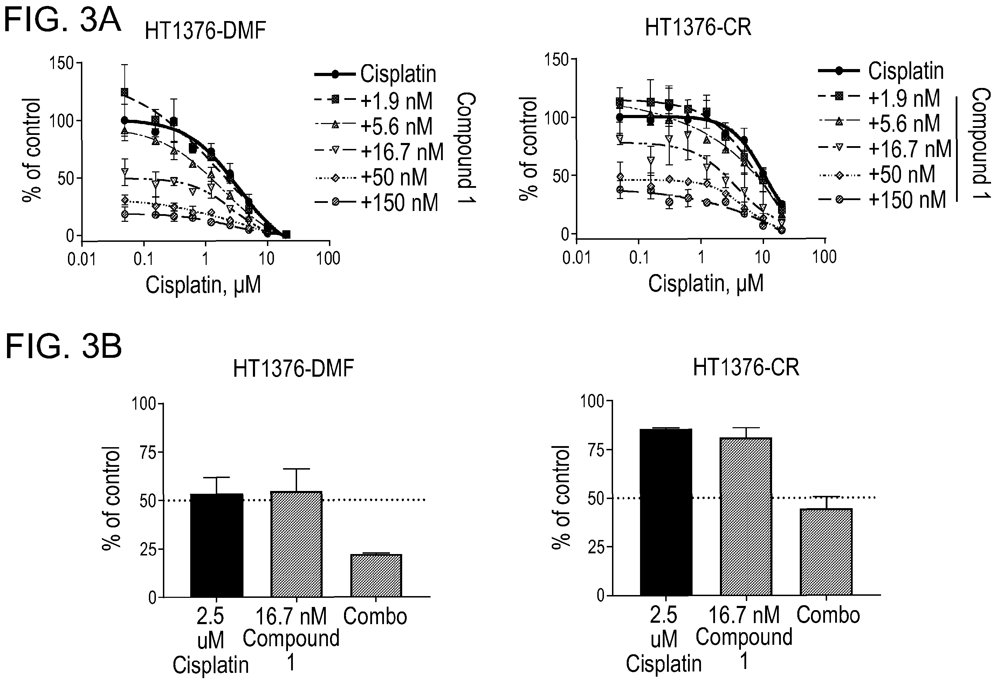

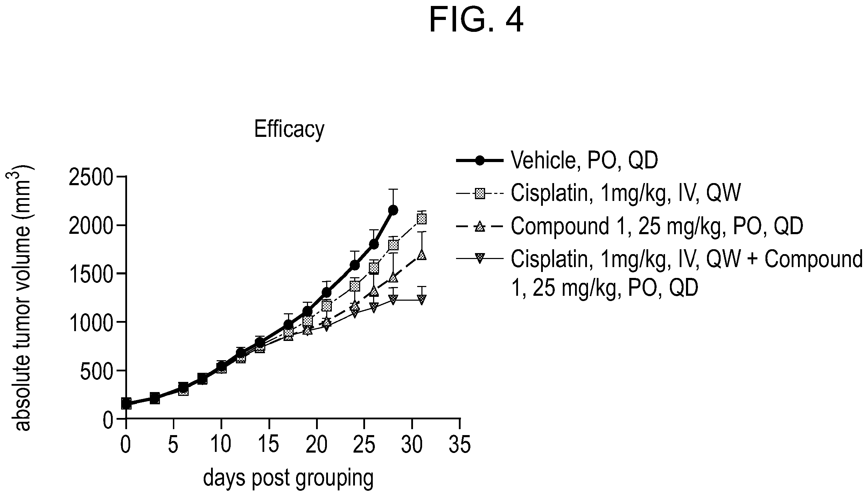

Similar results were seen in HT1376 bladder cancer cells lines. For example, ciplatin sensitive (-DMF) and resistant (-CR) HT1376 cell lines showed enhanced effects on cell growth when cisplatin treatment was combined with Compound **1.** See **FIG. 3A** and **FIG. 3B****.** In addition, Compound **1** alone and in combination with cisplatin was effective in reducing tumor growth. See **FIG. 4****.** Taken together, this data evidences that Compound **1** can be combined with other chemotherapeutic agents to synergistically treat solid tumors such as bladder and ovarian cancers.

### B. Synergy With Compound 1 and Enzalutamide (an Androgen receptor signaling inhibitor)

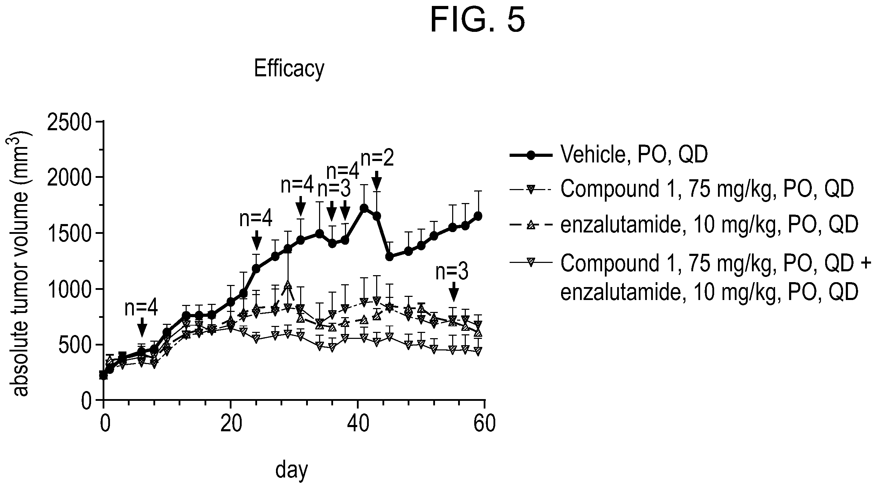

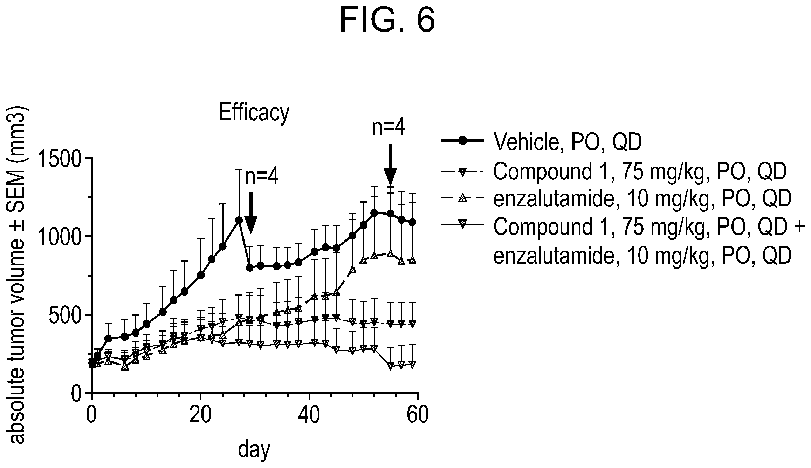

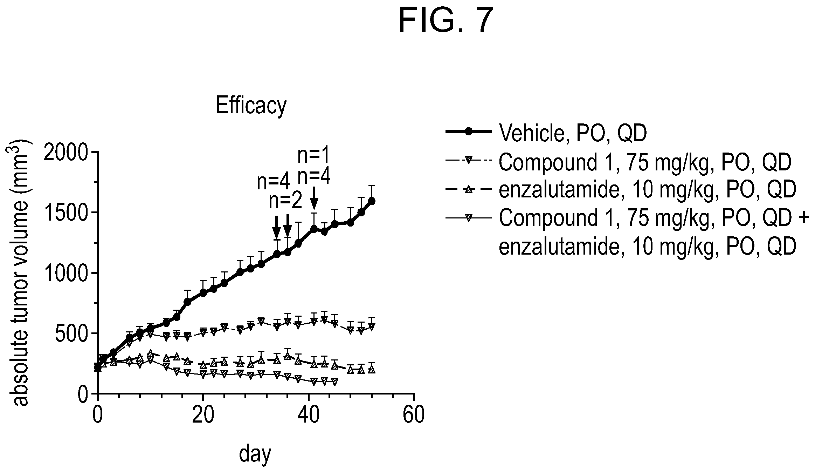

The antitumor effects of Compound **1** alone and in combination with the androgen receptor signaling inhibitor enzalutamide were evaluated in CTG 2428 PDX tumors in NOG mice. As shown by **FIG. 5****,** the combination of Compound **1** and enzalutamide reduces absolute tumor volume better than Compound **1** or enzalutamide alone. Similar results were seen in CTG-2440 PDX **(****FIG. 6****)** tumors and CTG-2441 PDX tumors in NOG mice **(****FIG. 7****).** This data establishes that Compound **1** can be combined with androgen receptor signaling inhibitors such as enzalutamide to treat solid tumors cancers such prostate cancer.

### 3. Primary In Vivo Pharmacology (Mono- and Combination Therapies)

A phase 1/2 Study to Evaluate the Safety, Tolerability, and Preliminary Clinical Activity of Compound **1** Monotherapy and in Combination with Irinotecan in 6 Disease-specific Dose Expansion Cohorts will be Conducted Following the General Procedures Outlined Below. Phase 1 will be composed of Compound **1** monotherapy dose escalation and combination therapy (Compound **1** + irinotecan) Dose Escalation periods in patients with advanced relapsed solid tumors; Phase 2 will include monotherapy dose expansion and combination therapy Dose Expansion periods in 6 disease-specific dose expansion cohorts.

### A. Single-Agent Efficacy

Patients enrolled in the following cohorts will receive oral Compound **1** monotherapy:
- Monotherapy Dose Escalation cohorts in patients with advanced relapsed solid tumors.
- Dose Expansion Cohort 1 in patients with urothelial carcinoma.
- Dose Expansion Cohort 2 in patients with ovarian clear cell carcinoma.
- Dose Expansion Cohort 3 in patients with endometrial carcinoma.

This study will enroll evaluable patients with advanced solid tumors across 2 phases. Eligibility will include certain criteria e.g., having relapsed following or progressed through standard therapy. Phase 1 is intended to determine the maximum tolerated dose (MTD) and/or recommended Phase 2 dose (RP2D) of Compound **1** as monotherapy in patients with advanced solid tumors. Secondary objectives include the safety and tolerability of Compound **1,** pharmacokinetic (PK) and pharmacodynamic (PD) profile of Compound **1,** and the preliminary clinical activity of Compound **1.** Phase 2 is designed to evaluate the antitumor activity of Compound **1** as monotherapy in patients with selected solid tumors (e.g., urothelial carcinoma, ovarian clear cell carcinoma, and endometrial carcinoma).

Patients enrolled in the monotherapy dose escalation portion of the study will receive Compound **1** orally (PO) once daily (QD) in continuous 4-week (28 days) cycles. The starting dose of Compound **1** is 50 mg. The Compound **1** dose will be escalated by ≤ 100% until at least 1 Grade 2 study drug related Adverse Event (except anemia or lymphocytopenia) is reported, after which the he Compound **1** dose may be escalated by ≤ 40%. Intermediate or additional dose levels may be evaluated if recommended based upon review of emerging safety, PK, or PD data. Compound **1** dose levels above 300 mg QD will be escalated by ≤ 25%.

### B. Combination Efficacy

Patients enrolled in the following cohorts will receive oral Compound **1** monotherapy:
- Combination Therapy Dose Escalation cohorts in patients with advanced relapsed solid tumors
- Dose Expansion Cohort 4 in patients with small cell lung cancer (SCLC)
- Dose Expansion Cohort 5 in patients with gastric or gastroesophageal junction (GEJ) adenocarcinoma
- Dose Expansion Cohort 6 in patients with serous ovarian cancer

This study will enroll evaluable patients with advanced solid tumors across the same 2 phases as the monotherapy dose, except that the selected solid tumors will be small cell lung cancer, gastric or gastroesophageal junction, and serous ovarian cancer. Eligibility will include certain criteria e.g., having relapsed following or progressed through standard therapy.

While have described a number of embodiments of this, it is apparent that our basic examples may be altered to provide other embodiments that utilize the compounds and uses of this disclosure. Therefore, it will be appreciated that the scope of this disclosure is to be defined by the appended claims rather than by the specific embodiments that have been represented by way of example.

Unless otherwise defined, all technical and scientific terms used herein are accorded the meaning commonly known to one with ordinary skill in the art.

## Claims

1. A compound having the formula: 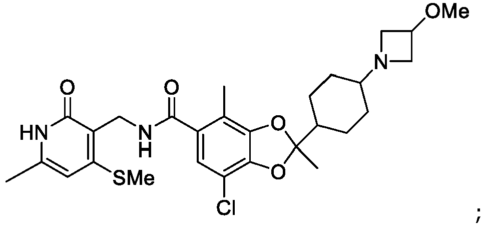 or a pharmaceutically acceptable salt thereof for use in the treatment of a solid tumor, wherein the treatment further comprises administration of an effective amount of second agent selected from a topoisomerase inhibitor, a DNA alkylating agent, and an androgen receptor signaling inhibitor.

2. The compound for use of Claim 1, wherein the second agent is an androgen receptor signaling inhibitor, such as bicalutamide, enzalutamide, apalutamide, flutamide, nilutamide, darolutamide, or abiraterone acetate.

3. The compound for use of Claim 1 or 2, wherein the second agent is enzalutamide.

4. The compound for use of Claim 1 or 2, wherein the second agent is darolutamide.

5. The compound for use of Claim 1 or 2, wherein the second agent is abiraterone acetate.

6. The compound for use of Claim 1, wherein the second agent is a DNA alkylating agent, such as busulfan, cyclophosphamide, bendmustine, carboplatin, chlorambucil, cyclophosphamide, cisplatin, temozolomide, melphalan, carmustine, lomustine, dacarbazine, oxaliplatin, ifosamide, thiotepa, trabectedin, altretamine, mechlorethamine, procarbazine, or streptozocin.

7. The compound for use of Claim 1 or 6, wherein the DNA alkylating agent is cisplatin.

8. The compound for use of Claim 1, wherein the second agent is a topoisomerase inhibitor, such as a topoisomerase I inhibitor, or wherein the topoisomerase inhibitor is selected from irinotecan, topotecan, camptothecin, lamellarin, etoposide, teniposide, doxorubicin, daunorubicin, mitoxantrone, amsacrine, ellipticines, aurintricarboxylic acid, HU-331, epirubicin, valrubicin, idarubicin, pixantrone, teniposide, belotecan, gimatecan, indotecan, and indimitecan.

9. The compound for use of Claim 1 or 8, wherein the topoisomerase inhibitor is irinotecan.

10. The compound for use of any one of Claims 1 to 9, wherein the solid tumor is selected from prostate cancer, small cell lung cancer (SCLC), gastric or gastroesophageal junction (GEJ) adenocarcinoma, and serous ovarian cancer.

11. The compound for use of any one of Claims 1 to 10, wherein the solid tumor is prostate cancer.

12. The compound for use of any one of Claims 1 to 3 and 6 to 10, wherein the solid tumor is metastatic castration-resistant prostate cancer.

13. The compound for use of any one of Claims 1 to 12, wherein the solid tumor is characterized as an advanced tumor.

14. The compound for use of any one of Claims 1 to 13, wherein the solid tumor is characterized as a relapsed solid tumor.

15. The compound for use of any one of Claims 1 to 14, wherein the compound is administered concurrently with the second agent.

16. The compound for use of any one of Claims 1 to 15, wherein the compound is of the formula 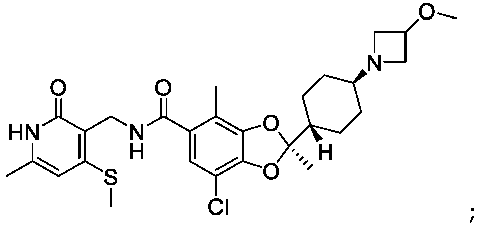 or a pharmaceutically acceptable salt thereof.

17. A compound having the formula: 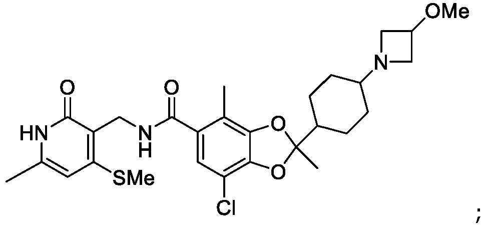 or a pharmaceutically acceptable salt thereof, for use in the treatment of an advanced relapsed solid tumor.

18. The compound for use of Claim 17, wherein the advanced relapsed solid tumor is advanced relapsed urothelial carcinoma, advanced relapsed ovarian clear cell carcinoma, or advanced relapsed endometrial carcinoma.

19. The compound for use of Claim 17 or 18, wherein the compound is of the formula: 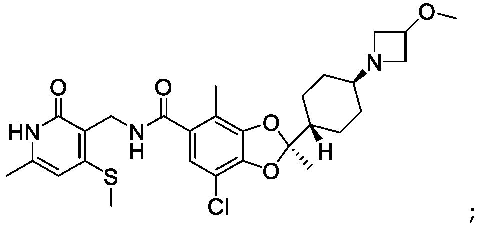 or a pharmaceutically acceptable salt thereof.

20. A compound having the formula: 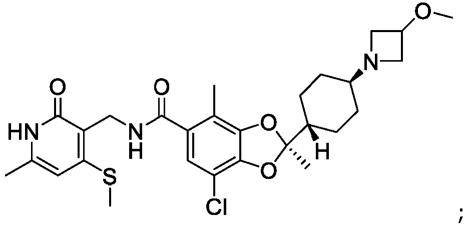 or a pharmaceutically acceptable salt thereof for use in the treatment of prostate cancer, wherein the treatment further comprises administration of an effective amount of enzalutamide.

21. A compound having the formula: 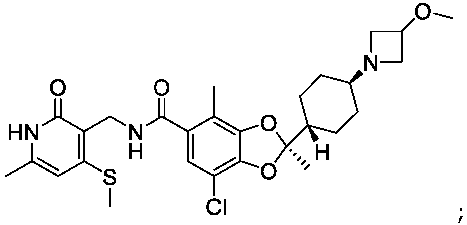 or a pharmaceutically acceptable salt thereof for use in the treatment of prostate cancer, wherein the treatment further comprises administration of an effective amount of darolutamide.

22. A compound having the formula: 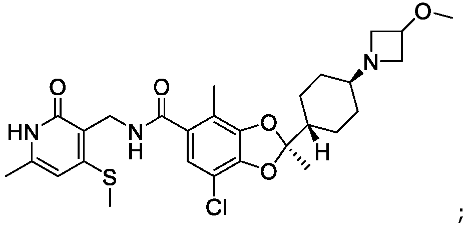 or a pharmaceutically acceptable salt thereof for use in the treatment of prostate cancer, wherein the treatment further comprises administration of an effective amount of abiraterone acetate.

23. The compound for use of Claim 20, wherein the prostate cancer is metastatic castration-resistant prostate cancer.

## Patentansprüche

1. Verbindung mit der Formel: 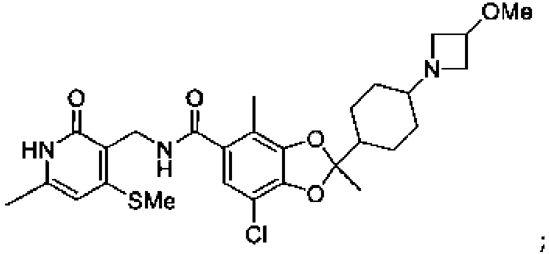 oder ein pharmazeutisch verträgliches Salz davon zur Verwendung bei der Behandlung eines soliden Tumors, wobei die Behandlung ferner die Verabreichung einer wirksamen Menge eines zweiten Mittels umfasst, ausgewählt aus einem Topoisomerase-Inhibitor, einem DNA-Alkylierungsmittel und einem Androgenrezeptor-Signalweginhibitor.

2. Verbindung zur Verwendung nach Anspruch 1, wobei das zweite Mittel ein Androgenrezeptor-Signalweginhibitor ist, wie Bicalutamid, Enzalutamid, Apalutamid, Flutamid, Nilutamid, Darolutamid oder Abirateronacetat.

3. Verbindung zur Verwendung nach Anspruch 1 oder 2, wobei das zweite Mittel Enzalutamid ist.

4. Verbindung zur Verwendung nach Anspruch 1 oder 2, wobei das zweite Mittel Darolutamid ist.

5. Verbindung zur Verwendung nach Anspruch 1 oder 2, wobei das zweite Mittel Abirateronacetat ist.

6. Verbindung zur Verwendung nach Anspruch 1, wobei das zweite Mittel ein DNA-Alkylierungsmittel ist, wie Busulfan, Cyclophosphamid, Bendmustin, Carboplatin, Chlorambucil, Cyclophosphamid, Cisplatin, Temozolomid, Melphalan, Carmustin, Lomustin, Dacarbazin, Oxaliplatin, Ifosamid, Thiotepa, Trabectedin, Altretamin, Mechlorethamin, Procarbazin oder Streptozocin.

7. Verbindung zur Verwendung nach Anspruch 1 oder 6, wobei das DNA-Alkylierungsmittel Cisplatin ist.

8. Verbindung zur Verwendung nach Anspruch 1, wobei das zweite Mittel ein Topoisomerase-Inhibitor ist, wie ein Topoisomerase-I-Inhibitor, oder wobei der Topoisomerase-Inhibitor ausgewählt ist aus Irinotecan, Topotecan, Camptothecin, Lamellarin, Etoposid, Teniposid, Doxorubicin, Daunorubicin, Mitoxantron, Amsacrin, Ellipticinen, Aurintricarbonsäure, HU-331, Epirubicin, Valrubicin, Idarubicin, Pixantron, Teniposid, Belotecan, Gimatecan, Indotecan und Indimitecan.

9. Verbindung zur Verwendung nach Anspruch 1 oder 8, wobei der Topoisomerase-Inhibitor Irinotecan ist.

10. Verbindung zur Verwendung nach einem der Ansprüche 1 bis 9, wobei der solide Tumor ausgewählt ist aus Prostatakrebs, kleinzelligem Lungenkrebs (SCLC), Adenokarzinom des Magens oder des gastroösophagealen Übergangs (GEJ) und serösem Eierstockkrebs.

11. Verbindung zur Verwendung nach einem der Ansprüche 1 bis 10, wobei der solide Tumor Prostatakrebs ist.

12. Verbindung zur Verwendung nach einem der Ansprüche 1 bis 3 und 6 bis 10, wobei der solide Tumor metastasierter kastrationsresistenter Prostatakrebs ist.

13. Verbindung zur Verwendung nach einem der Ansprüche 1 bis 12, wobei der solide Tumor durch einen fortgeschrittenen Tumor **gekennzeichnet** ist.

14. Verbindung zur Verwendung nach einem der Ansprüche 1 bis 13, wobei der solide Tumor durch einen rezidivierten soliden Tumor **gekennzeichnet** ist.

15. Verbindung zur Verwendung nach einem der Ansprüche 1 bis 14, wobei die Verbindung gleichzeitig mit dem zweiten Mittel verabreicht wird.

16. Verbindung nach einem der Ansprüche 1 bis 15, wobei die Verbindung die Formel aufweist 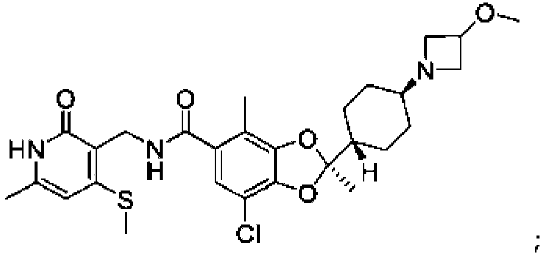 oder ein pharmazeutisch verträgliches Salz davon ist.

17. Verbindung mit der Formel: 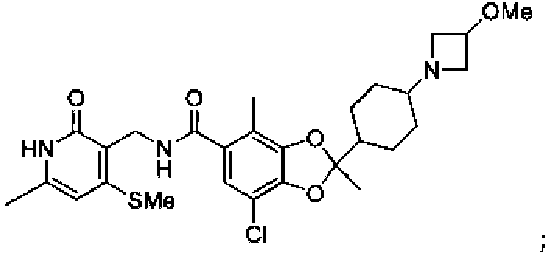 oder ein pharmazeutisch verträgliches Salz davon, zur Verwendung bei der Behandlung eines fortgeschrittenen rezidivierten soliden Tumors.

18. Verbindung zur Verwendung nach Anspruch 17, wobei der fortgeschrittene rezidivierte solide Tumor ein fortgeschrittenes rezidiviertes Urothelkarzinom, ein fortgeschrittenes rezidiviertes klarzelliges Ovarialkarzinom oder ein fortgeschrittenes rezidiviertes Endometriumkarzinom ist.

19. Verbindung zur Verwendung nach Anspruch 17 oder 18, wobei die Verbindung die Formel aufweist: 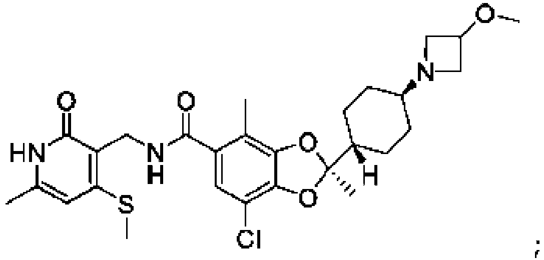 oder ein pharmazeutisch verträgliches Salz davon ist.

20. Verbindung mit der Formel: 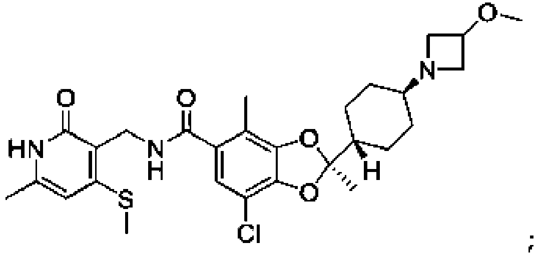 oder ein pharmazeutisch verträgliches Salz davon zur Verwendung bei der Behandlung von Prostatakrebs, wobei die Behandlung ferner die Verabreichung einer wirksamen Menge an Enzalutamid umfasst.

21. Verbindung mit der Formel: 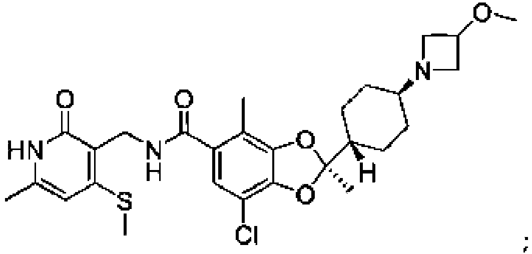 oder ein pharmazeutisch verträgliches Salz davon zur Verwendung bei der Behandlung von Prostatakrebs, wobei die Behandlung ferner die Verabreichung einer wirksamen Menge von Darolutamid umfasst.

22. Verbindung mit der Formel: 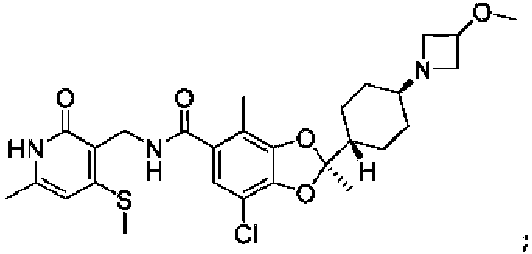 oder ein pharmazeutisch verträgliches Salz davon zur Verwendung bei der Behandlung von Prostatakrebs, wobei die Behandlung ferner die Verabreichung einer wirksamen Menge von Abirateronacetat umfasst.

23. Verbindung zur Verwendung nach Anspruch 20, wobei der Prostatakrebs metastasierter kastrationsresistenter Prostatakrebs ist.

## Revendications

1. Composé ayant la formule : 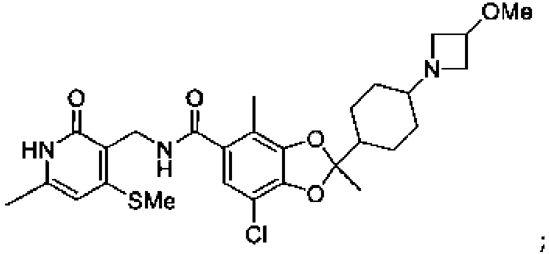 ou un sel pharmaceutiquement acceptable de celui-ci pour une utilisation dans le traitement d'une tumeur solide, dans lequel le traitement comprend en outre l'administration d'une quantité efficace d'un second agent choisi parmi un inhibiteur de topoisomérase, un agent alkylant de l'ADN, et un inhibiteur de la signalisation du récepteur des androgènes.

2. Composé pour une utilisation selon la revendication 1, dans lequel le second agent est un inhibiteur de la signalisation du récepteur des androgènes, tel que bicalutamide, enzalutamide, apalutamide, flutamide, nilutamide, darolutamide, ou acétate d'abiratérone.

3. Composé pour une utilisation selon la revendication 1 ou 2, dans lequel le second agent est l'enzalutamide.

4. Composé pour une utilisation selon la revendication 1 ou 2, dans lequel le second agent est le darolutamide.

5. Composé pour une utilisation selon la revendication 1 ou 2, dans lequel le second agent est l'acétate d'abiratérone.

6. Composé pour une utilisation selon la revendication 1, dans lequel le second agent est un agent alkylant de l'ADN, tel que busulfan, cyclophosphamide, bendamustine, carboplatine, chlorambucil, cyclophosphamide, cisplatine, témozolomide, melphalan, carmustine, lomustine, dacarbazine, oxaliplatine, ifosfamide, thiotépa, trabectédine, altrétamine, méchloréthamine, procarbazine, ou streptozocine.

7. Composé pour une utilisation selon la revendication 1 ou 6, dans lequel l'agent alkylant de l'ADN est le cisplatine.

8. Composé pour une utilisation selon la revendication 1, dans lequel le second agent est un inhibiteur de topoisomérase, tel qu'un inhibiteur de topoisomérase I, ou dans lequel l'inhibiteur de topoisomérase est choisi parmi irinotécan, topotécan, camptothécine, lamellarine, étoposide, téniposide, doxorubicine, daunorubicine, mitoxantrone, amsacrine, ellipticines, acide aurintricarboxylique, HU-331, épirubicine, valrubicine, idarubicine, pixantrone, téniposide, bélotécan, gimatécan, indotécan, et indimitécan.

9. Composé pour une utilisation selon la revendication 1 ou 8, dans lequel l'inhibiteur de topoisomérase est l'irinotécan.

10. Composé pour une utilisation selon l'une quelconque des revendications 1 à 9, dans lequel la tumeur solide est choisie parmi cancer de la prostate, cancer du poumon à petites cellules (SCLC), adénocarcinome gastrique ou de la jonction gastro-œsophagienne (GEJ), et cancer séreux de l'ovaire.

11. Composé pour une utilisation selon l'une quelconque des revendications 1 à 10, dans lequel la tumeur solide est un cancer de la prostate.

12. Composé pour une utilisation selon l'une quelconque des revendications 1 à 3 et 6 à 10, dans lequel la tumeur solide est un cancer de la prostate métastatique résistant à la castration.

13. Composé pour une utilisation selon l'une quelconque des revendications 1 à 12, dans lequel la tumeur solide est caractérisée comme étant une tumeur avancée.

14. Composé pour une utilisation selon l'une quelconque des revendications 1 à 13, dans lequel la tumeur solide est caractérisée comme étant une tumeur solide de récidive.

15. Composé pour une utilisation selon l'une quelconque des revendications 1 à 14, dans lequel le composé est administré conjointement au second agent.

16. Composé pour une utilisation selon l'une quelconque des revendications 1 à 15, dans lequel le composé est de la formule 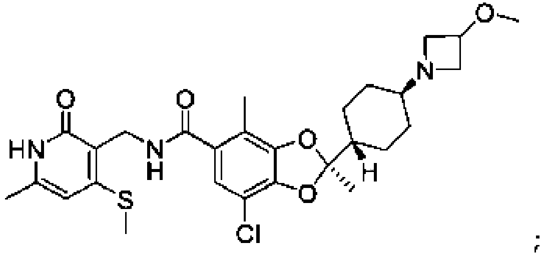 ou un sel pharmaceutiquement acceptable de celui-ci.

17. Composé ayant la formule : 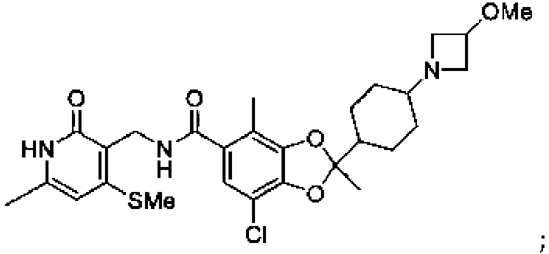 ou un sel pharmaceutiquement acceptable de celui-ci, pour une utilisation dans le traitement d'une tumeur solide de récidive avancée.

18. Composé pour une utilisation selon la revendication 17, dans lequel la tumeur solide de récidive avancée est un carcinome urothélial récidivant avancé, un carcinome ovarien à cellules claires récidivant avancé, ou un carcinome de l'endomètre récidivant avancé.

19. Composé pour une utilisation selon la revendication 17 ou 18, dans lequel le composé est de la formule : 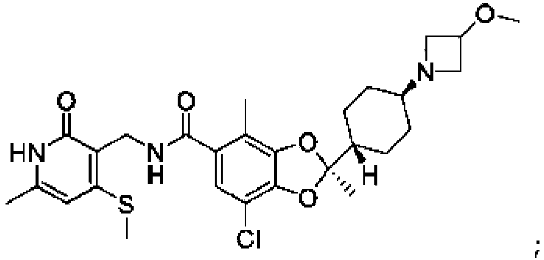 ou un sel pharmaceutiquement acceptable de celui-ci.

20. Composé ayant la formule : 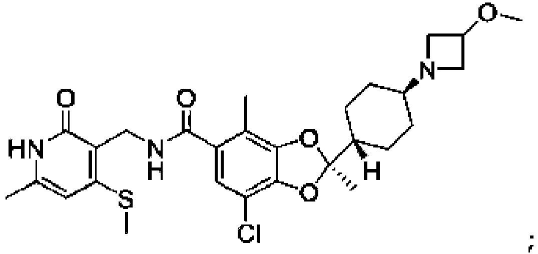 ou un sel pharmaceutiquement acceptable de celui-ci pour une utilisation dans le traitement du cancer de la prostate, dans lequel le traitement comprend en outre l'administration d'une quantité efficace d'enzalutamide.

21. Composé ayant la formule : 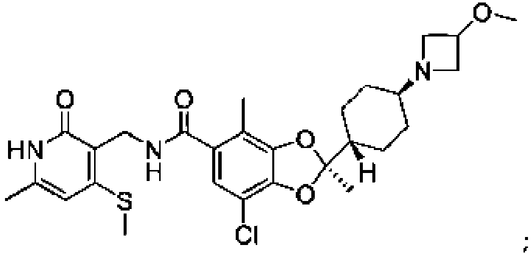 ou un sel pharmaceutiquement acceptable de celui-ci pour une utilisation dans le traitement du cancer de la prostate, dans lequel le traitement comprend en outre l'administration d'une quantité efficace de darolutamide.

22. Composé ayant la formule : 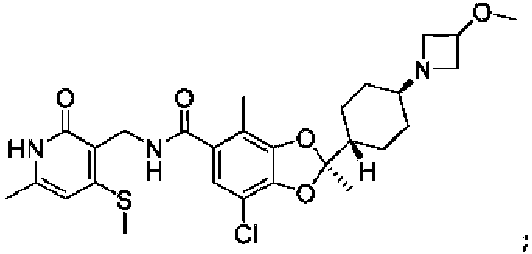 ou un sel pharmaceutiquement acceptable de celui-ci pour une utilisation dans le traitement du cancer de la prostate, dans lequel le traitement comprend en outre l'administration d'une quantité efficace d'acétate d'abiratérone.

23. Composé pour une utilisation selon la revendication 20, dans lequel le cancer de la prostate est un cancer de la prostate métastatique résistant à la castration.
